Europäisches Patentamt

European Patent Office

Office européen des brevets

(19)

(11) Numéro de publication: **0 017 559**

A1

(12) **DEMANDE DE BREVET EUROPEEN**

(21) Numéro de dépôt: **80400397.8**

(22) Date de dépôt: **26.03.80**

(51) Int. Cl.³: **C 07 C 79/02**
**B 01 J 3/04**

(30) Priorité: **28.03.79 FR 7907840**

(43) Date de publication de la demande:
**15.10.80 Bulletin 80/21**

(84) Etats Contractants Désignés:
**AT BE CH DE FR GB IT LU NL SE**

(71) Demandeur: **SOCIETE CHIMIQUE DE LA GRANDE PAROISSE AZOTE ET PRODUITS CHIMIQUES**
**8, rue Cognacq-Jay**
**F-75321 Paris Cedex 07(FR)**

(72) Inventeur: **Lhonore, Pierre**
**1090, bd Jeanne d'Arc**
**F-59500 Douai(FR)**

(72) Inventeur: **Quibel, Jacques**
**40, rue de la Muette**
**F-78600 Maisons Laffitte(FR)**

(72) Inventeur: **Jacquinot, Bernard**
**59, rue Léon Bathiat**
**F-59500 Douai(FR)**

(74) Mandataire: **Bouton Neuvy, Liliane et al,**
**L'Air liquide, Société Anonyme pour L'Etude et L'Exploitation des Procédés Georges Claude 75, Quai d'Orsay**
**F-75321 Paris Cedex 07(FR)**

(54) Réacteur de nitration d'hydrocarbures en phase gazeuse sous pression.

(57) La présente invention concerne un réacteur de nitration d'hydrocarbures en phase gazeuse sous pression.

Le réacteur est constitué par une enceinte réactionnelle à l'intérieur de laquelle un faisceau tubulaire est en contact avec un fluide caloporteur de forte capacité d'échange thermique.

Le réacteur est utilisé avantageusement dans la nitration des hydrocarbures saturés inférieurs à $C_5$, seuls ou en mélange.

EP 0 017 559 A1

Croydon Printing Company Ltd.

SOCIETE CHIMIQUE DE LA GRANDE PAROISSE, AZOTE ET PRODUITS CHIMIQUES.

"REACTEUR DE NITRATION D'HYDROCARBURES EN PHASE GAZEUSE SOUS PRESSION"

La présente invention concerne un réacteur de nitration d'hydrocarbures en phase gazeuse sous pression.

Il a déjà été proposé divers procédé de nitration des hydrocarbures. La demanderesse a particulièrement étudié la nitration du propane, de l'éthane et des mélanges de ces hydrocarbures.

Le brevet d'invention France 71.38964, publié sous le numéro 2.158.708 au nom de la déposante décrit la nitration du propane par le peroxyde d'azote en présence d'oxygène, introduit sous forme d'air, sous une pression de 8 à 14 bars et à des températures d'introduction des réactants dans la zone réactionnelle de l'ordre de 200 à 240° C.

La demande de brevet européenne 4.812 du 28 mars 1979 également au nom de la déposante est relative à la nitration en phase gazeuse sous pression d'un mélange contenant une quantité substantielle de propane. Les température et pression réactionnelle, le temps de contact ainsi que les rapports quantitatifs entre l'agent nitrant, le mélange à nitrer et le gaz oxygéné sont choisis de manière telle que la réaction de nitration soit mise en oeuvre en phase gazeuse homogène. Le mélange à nitrer contient du propane et un ou plusieurs autres alcanes ayant jusqu'à cinq atomes de carbone dans la molécule.

Les conditions de nitration de l'éthane ont fait l'objet de la demande européenne déposée le 9 novembre 1979 sous le n° 794008359 aussi au nom de la Société Chimique de la Grande Paroisse. Selon ce procédé les rapports quantitatifs des divers constituants du mélange réactionnel, le temps de contact réactionnel, les températures et la pression réactionnelles sont choisis et contrôlés de manière telle que la nitration de l'éthane soit mise en oeuvre en phase gazeuse homogène, et en fonction du spectre attendu des nitroparaffines.

Comme dans les précédents procédés de nitration du propane ou de mélanges à base de propane, la réaction de nitration peut être conduite en présence d'un agent actif porteur d'un groupement NO ou $NO_2$ facilement transférable, tel le 2-nitropropane ou le nitroéthane seuls ou en mélange, éventuellement produit réactionnel recyclé. Ces nitrations sont aussi avantageusement mises en oeuvre en présence de gaz inerte, choisi parmi l'azote, l'oxyde de carbone, le dioxyde de carbone, l'hydrogène, le méthane, l'argon ou un mélange quelconque de ces gaz.

Pour tous ces procédés on obtient les rendements les plus élevés qu'en travaillant en régime thermique progressif et régulier, c'est-à-dire en contrôlant la courbe de température à l'intérieur de la zone réactionnelle ; cette courbe devant avoir une croissance régulière sans points d'inflexion. Un tel régime thermique ne peut être obtenu qu'en évitant tout emballement de la réaction et l'apparition de températures élevées en certains points de la zone réactionnelle.

Il a été trouvé un type de réacteur utilisable dans toutes les nitrations d'hydrocarbures en phase gazeuse sous pression. Ce réacteur par sa structure permet l'obtention d'un régime thermique à progression régulière au cours de la réaction de nitration, c'est-à-dire que l'évolution de la courbe de température de la réaction est continue et progressive, sans accélération brusque de la vitesse réactionnelle et sans apparition de pointes de température en divers endroits de la zone réactionnelle. Ce réacteur est spécialement conçu pour avoir un bon échange de chaleur et une maitrise de la courbe d'élévation de température pendant la réaction.

Le réacteur selon l'invention est constitué par une enceinte réactionnelle à l'intérieur de laquelle un faisceau tubulaire est en contact avec un fluide caloporteur de forte capacité d'échange thermique. Le fluide caloporteur qui véhicule la chaleur de la réaction de nitration ou celle nécessaire à l'échauffement des réactants peut être de la vapeur, un bain de sels fondus ou tout fluide caloporteur approprié. Le fluide caloporteur est choisi de manière telle que la température de la peau extérieure des tubes soit maintenue constante à 20° C près entre le point le plus chaud et le point le plus froid de ceux-ci.

Le plus souvent le faisceau tubulaire est plongé dans le fluide caloporteur et la réaction de nitration a lieu à l'intérieur des tubes. On peut envisager de mettre la nitration en oeuvre à l'extérieur des tubes, le fluide caloporteur étant logé à l'intérieur des tubes.

Le dimensionnement des tubes du faisceau joue un rôle dans la croissance régulière de la température pendant l'évolution de la réaction. La forme de la section des tubes est indifférente si elle permet le bon écoulement des fluides gazeux, soit circulaire ou ovale, à condition que le périmètre intérieur des tubes du faisceau soit au plus égal à 800 millimètres, le diamètre intérieur des tubes de section circulaire étant au plus égal à 250 millimètres.

De plus, on a remarqué que le choix judicieux du rapport entre la surface du faisceau tubulaire en contact avec le milieu réactionnel et le volume de l'enceinte réactionnelle a une influence favorable sur la croissance régulière de la température au cours de la réaction, quand ce rapport est compris entre 0,1 et 20, de préférence 0,5 et 5, et en particulier entre 1 et 3.

L'obtention de produits de nitration avec de bons rendements est aussi en relation avec le maintien régulier des temps de contact dans tous les tubes du faisceau, ceci n'est d'ailleurs valable qu'avec des débits comparables dans les divers tubes du faisceau. La répartition régulière du débit dans les divers tubes du faisceau peut être assurée par un moyen mécanique dont est muni le réacteur. Il est avantageux de répartir le débit de manière telle que la différence maximale de charge entre le tube le plus chargé et le tube le moins chargé soit au plus égale à 10 %.

Le réacteur est alimenté par un injecteur qui assure un mélange homogène de l'ensemble des fluides réactionnels.

Le faisceau tubulaire est construit en métal résistant à la corrosion de l'agent nitrant : acide nitrique, peroxyde d'azote seuls ou en mélange ou tout agent porteur d'un groupe NO ou $NO_2$ facilement transférable. Et le couple métallique formé avec le métal de l'injecteur est rigoureusement choisi de manière telle qu'il ne provoque pas l'allumage des fluides réactionnels à l'entrée de la chambre de réaction.

Le réacteur de nitration précédemment décrit est utilisé avec d'excellentes performances dans tous les types de nitration des hydrocarbures en phase gazeuse sous pression, notamment des hydrocarbures saturés inférieurs à $C_5$, en particulier de l'éthane et du propane et de leurs mélanges, éventuellement en présence d'oxygène, de produits de recyclage, d'adjuvants et de gaz inertes.

Les procédés décrits dans les brevets précédemment cités sont avantageusement mis en oeuvre dans le réacteur objet de l'invention.

REVENDICATIONS

1. Réacteur de nitration d'hydrocarbures en phase gazeuse, sous pression constitué par une enceinte réactionnelle à l'intérieur de laquelle un faisceau tubulaire est en contact avec un fluide caloporteur de forte capacité d'échange thermique, caractérisé en ce que le fluide caloporteur est choisi de manière telle que la température de la peau extérieure des tubes soit maintenue constante à 20° C près entre le point le plus chaud et le point le plus froid, le rapport de la surface du faisceau tubulaire en contact avec le milieu réactionnel au volume de l'enceinte réactionnelle étant compris entre 0,1 et 20, et le périmètre intérieur des tubes du faisceau étant au plus égal à 800 mm.

2. Réacteur de nitration en phase gazeuse selon la revendication 1, caractérisé en ce que le diamètre intérieur des tubes de section circulaire est au plus égal à 250 mm.

3. Réacteur de nitration en phase gazeuse selon la revendication 1, caractérisé en ce que le rapport de la surface du faisceau tubulaire en contact avec le milieu réactionnel au volume de l'enceinte réactionnelle est compris entre 0,5 et 5.

4. Réacteur de nitration en phase gazeuse selon la revendication 1, caractérisé en ce que le rapport de la surface du faisceau tubulaire en contact avec le milieu réactionnel au volume de l'enceinte réactionnelle est compris entre 1 et 3.

5. Réacteur de nitration d'hydrocarbures en phase gazeuse sous pression, selon la revendication 1, caractérisé en ce que le réacteur est muni d'un moyen mécanique qui répartit régulièrement le débit dans les différents tubes du faisceau de manière telle que la différence maximale de charge entre le tube le plus chargé et le tube le moins chargé soit au plus égale à 10 %.

6. Réacteur de nitration d'hydrocarbures en phase gazeuse sous pression selon la revendication 1, caractérisé en ce que le faisceau tubulaire est alimenté par un injecteur qui assure un mélange homogène de l'ensemble des fluides réactionnels.

7. Réacteur de nitration d'hydrocarbures en phase gazeuse dont le faisceau tubulaire est en métal résistant à la corrosion de l'agent nitrant et des oxydes d'azote selon la revendication 6, caractérisé en ce que le couple formé entre le métal du faisceau tubulaire et celui de l'injecteur est choisi de manière telle qu'il ne provoque pas l'allumage des fluides réactionnels à l'entrée à l'enceinte réactionnelle.

8. Réacteur de nitration d'hydrocarbures en phase gazeuse selon la revendication 1, caractérisé en ce qu'il est utilisé dans la nitration des hydrocarbures saturés inférieurs à $C_5$ tels l'éthane et le propane et de leurs mélanges, éventuellement en présence de produits de recyclage, d'adjuvants et de gaz inertes.

Office européen
des brevets

**RAPPORT DE RECHERCHE EUROPEENNE**

| DOCUMENTS CONSIDERES COMME PERTINENTS | | | CLASSEMENT DE LA DEMANDE (Int. Cl. ³) |
|---|---|---|---|
| Catégorie | Citation du document avec indication en cas de besoin des parties pertinentes | Revendication concernee | |
| X | US - A - 2 161 475 (G.K. LANDON) <br> * Page 1, colonne 1, lignes 1,2; page 1, colonne 2, lignes 3-40; page 2, colonne 1, lignes 9-67; revendications; figure * <br><br> -- | 1,3,4, 6,8 | C 07 C 79/02 <br> B 01 J 3/04 |
| | US - A - 2 164 774 (G.K. LANDON) <br> * Page 1, colonne 1, lignes 1,2; page 1, colonne 2, ligne 7 - page 2, colonne 1, ligne 13; figure * <br><br> -- | 1,6,8 | **DOMAINES TECHNIQUES RECHERCHES (Int. Cl. ³)** |
| A | US - A - 2 512 587 (L.A. STENGEL) <br> * Colonne 1, lignes 1-8; colonne 3, lignes 28-34; colonne 3, ligne 39 - colonne 4, ligne 32; exemple I; figure I * <br><br> -- | 1,8 | B 01 J 3/04 <br> C 07 C 79/02 <br> 79/04 |
| A | US - A - 2 511 454 (R.B. BISHOP et al.) <br> * Colonne 1, lignes 1-7; colonne 2, ligne 40 - colonne 3, ligne 21; colonne 3, ligne 38 - colonne 5, ligne 49 * <br><br> -- | 1,8 | |
| A | FR - A - 962 276 (SOCONY-VACUUM OIL) | 1,8 | **CATEGORIE DES DOCUMENTS CITES** <br><br> X: particulièrement pertinent <br> A: arrière-plan technologique <br> O: divulgation non-écrite <br> P: document intercalaire |
| D,A | FR - A - 2 158 708 (SOC. CHIM. DE LA GRANDE PAROISSE) | | T: théorie ou principe à la base de l'invention <br> E: demande faisant interference |
| D,A, P | EP - A - 0 004 812 (SOC. CHIM. DE LA GRANDE PAROISSE) (date de dépôt 28-3-1979) (publié 17-10-1979) <br><br> ./.. | | D: document cite dans la demande <br> L: document cite pour d autres raisons <br><br> &: membre de la même famille. document correspondant |

☒ Le present rapport de recherche a ete etabli pour toutes les revendications

| Lieu de la recherche <br> La Haye | Date d'achevement de la recherche <br> 08-07-1980 | Examinateur <br> SIEM |
|---|---|---|

OEB Form 1503.1   06.78

**Office européen des brevets**

**RAPPORT DE RECHERCHE EUROPEENNE**

## DOCUMENTS CONSIDERES COMME PERTINENTS

| Catégorie | Citation du document avec indication, en cas de besoin, des parties pertinentes | Revendication concernée |
|---|---|---|
| D,A | EP - A - 0 011 553 (SOC. CHIM. DE LA GRANDE PAROISSE) (date de dépôt 9-11-1979) (date de publication 28-5-1980)<br><br>---- | |

**CLASSEMENT DE LA DEMANDE (Int. Cl. 3)**

**DOMAINES TECHNIQUES RECHERCHES (Int. Cl. 3)**

OEB Form 1503.2   06.78